# EUROPEAN PATENT SPECIFICATION

(11) **EP 2 811 922 B1**
(45) Date of publication and mention of the grant of the patent: **27.04.2016**
(21) Application number: 13705360.9
(22) Date of filing: 08.02.2013
(51) Int. Cl.: A61B 17/32, A61B 17/16, A61B 17/3207

(54) **TISSUE RESECTION DEVICE**
GEWEBERESEKTIONSVORRICHTUNG
DISPOSITIF DE RÉSECTION DE TISSUS

(30) Priority: 08.02.2012 US 201261596590 P; 04.06.2012 US 201261655391 P; 11.07.2012 US 201261670540 P; 06.11.2012 US 201261722940 P
(43) Date of publication of application: 17.12.2014
(73) Proprietor: IMDS Corporation, Logan, UT 84321 (US); Kather, Jens, 8121 Benglen (CH); Schueler, Michael, 8280 Kreuszlingen (CH)
(72) Inventor: KATHER, Jens, CH-8121 Benglen (CH); JUSTIN, Daniel F., Orlando, FL 32817 (US); BUTTERS, Joshua A., Chandler, AZ 85224 (US); ARNETT, Jeffery D., Gilbert, AZ 85297 (US); SLATER, Nicholas, Chandler, AZ 85248 (US); HUSHKA, Dylan M., Gilbert, AZ 85296 (US); SCOTT, Melissa D., Flanders, NJ 07836 (US); COALE, Bradford J., Flanders, NJ 07836 (US)
(74) Representative: Dienwiebel, Thomas
(86) International application number: PCT/US2013/025414
(87) International publication number: WO 2013/120004

(56) References cited:
- EP-A1- 1 389 458
- WO-A2-2004/075719
- US-A- 5 569 284
- US-A1- 2005 203 508
- US-A1- 2010 179 557
- US-A1- 2011 004 215
- US-A1- 2011 196 399
- US-A1- 2013 023 882

## Description

The present disclosure relates to surgical instruments, such as arthroscopic surgical instruments. The principles herein are applicable in tissue removal applications, whether arthroscopic, laparoscopic, endoscopic, or open, including but not limited to: foot, ankle, knee, hip, pelvis, spine, ribs, shoulder, elbow, wrist, hand, craniomaxillofacial, etc.

Straight or spherical rigid cutting instruments are not well suited to creating a smooth anatomic radius of curvature with minimal manipulation. Therefore, with these tools, it is up to the surgeon to sculpt a three-dimensional (3D) anatomic surface by manipulating the cutter over the treated surface, without unintentionally removing too much tissue. This requires great skill, practice, patience, and time.

Document EP 1389458 A1, on which the preamble of claim 1 is based, discloses a medical rinsing and sucking device, comprising a connection tube, having a basal end portion connected with a waste matter sucking tube communication with a waste matter tank provided with a sucking pump and a front end portion connected with a rostral sucking nozzle for drawing the waste water, and a jet nozzle for jetting the rinsing water, having a basal end portion connected with a rinsing liquid tube communication with a rinsing water tank provided with a compressor, wherein the said connection tube and said jet nozzle are installed making the respective front ends thereof orient substantially the idential directions.

Document US 2011/0196399 A1 discloses an ultrasonic surgical instrument comprising a housing; an ultrasonic transducer assembly rotatably supported within said housing and communicating with a source of ultrasonic electrical signals; a motor within said housing and communicating with a source of motor drive signals, said motor communicating with said ultrasonic transducer assembly for applying rotational motion thereto; a horn coupled to said ultrasonic transducer assembly; a hollow sheath coupled to said housing and having at least one access window therein; a source of vacuum communicating with said hollow sheath for drawing tissue into said at least one access window; and a blade coupled to said horn and extending within said hollow sheath, said blade having at least one distal tissue cutting portion thereon arranged to interact with tissue drawn through said at least one access window.

Document US 2005/0203508 A1 discloses an alignment guide for alignment with an anatomical structure, comprising a pattern arm having a geometry tracking the anatomical structure; and a reference element connected to said pattern arm, said reference element fixable relative to the anatomical structure.

It is desirable to provide a more efficient means of tissue removal, including removal of sclerotic bone, in order to reduce operating time. The disclosed examples are capable of removing tissue on multiple surfaces at once, creating a smooth uniform surface with minimal manipulation of the instrument. The instruments described herein may automatically re-establish a proper anatomic profile to the treated surface by matching the natural anatomic profile of the tissue. The instruments described herein are capable of producing 3D shaping with simple two-dimensional (2D) manipulation of the instruments. The instruments and methods described herein may significantly reduce operating time and produce more uniform results.

The tissue resection devices or burr tools disclosed herein are capable of matching the natural anatomic curvature of a tissue. In one example, the tissue resection device includes an outer housing, a drive member, a central flexible member, rotary cutting elements, and bearing elements. The outer housing and/or cutting elements may be curved to approximately match the geometry of a tissue. The outer housing or sheath may have at least one outout, opening, or window through which the cutting elements, inserts, or burrs are exposed to effect the tissue resection. The cutout or window in the outer housing may be sized, shaped, and/or adjusted to vary the amount of burr exposure through the window to vary the amount/depth of tissue that is removed in a single pass of the instrument. In this manner, the window may act like a depth stop to provide extra control and precision over tissue removal and prevent unintended tissue removal. In some examples, the depth stop may allow a substantially uniform depth cut along the curved burr portion of the resection device. For example, the window in the outer sheath may be sized to allow the burrs to project about 1 mm from the window. In other examples, the burrs may project more or less than 1 mm. In yet other examples, the user may selectively adjust how much the burrs project from the window. In this manner, the user may control the depth of the tissue cut in a single pass and prevent the resection device from cutting away too much tissue.

In some examples disclosed herein, the cutting inserts, elements, or burrs are circular members that include a number of cutting arms with sharp edges that are designed to cut tissue, such as bone. The number of cutting arms may be varied to remove more or less tissue in a single revolution of the burr. In one example, the cutting insert has three arms. The cutting elements may also include a central hole through which a central flexible member may pass. The burrs may be slid over the central flexible member to create a flexible cutting assembly. In this example, the burrs may act like beads on a necklace moving and flexing with the central member. In some examples, the central flexible member may, or may not, be required to provide torque to the system as the burrs themselves may transmit the torque required for cutting.

In some applications of the disclosed technology, the efficacy of the tissue removal procedure may be evaluated by removing all instruments and articulating the affected joint post-resection to ensure impingement-free motion. Direct visualization, medical imaging, palpation, or other assessment means may also be used to evaluate the effectiveness of the tissue removal. Operative time may be further reduced by using measurement devices that serve as anatomic templates for the treated region.

### BRIEF DESCRIPTION OF THE DRAWINGS

While examples of the present technology have been shown and described in detail below, it will be clear to the person skilled in the art that variations, changes and modifications may be made without departing from its scope. As such, that which is set forth in the following description and accompanying drawings is offered by way of illustration only and not as a limitation. The actual scope of the invention is intended to be defined by the following claims, along with the full range of equivalents to which such claims are entitled.

In the following Detailed Description, various features are grouped together in several examples for the purpose of streamlining the disclosure. This method of disclosure is not to be interpreted as reflecting an intention that examples of the technology require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example.

Identical reference numerals do not necessarily indicate an identical structure. Rather, the same reference numeral may be used to indicate a similar feature or a feature with similar functionality. Not every feature of each example is labeled in every figure in which that example appears, in order to keep the figures clear. Similar reference numbers (e.g., those that are identical except for the first numeral) are used to indicate similar features in different examples.
FIG. 1 is an isometric view of a tissue resection device;
FIG. 2 is a side view of the tissue resection device of FIG. 1 ;
FIG. 3 is a bottom view of the tissue resection device of FIG. 1 ;
FIG. 4 is an isometric view of the tissue resection device of FIG. 1 with a hollow member removed;
FIG. 5 is an isometric view of another tissue resection device which incorporates an outer sheath with a protective hood;
FIG. 6 is a side view of the tissue resection device of FIG. 5 with the protective hood positioned in a first position;
FIG. 7 is a side view of the tissue resection device of FIG. 5 with the protective hood positioned in a second position;
FIG. 8 is a close-up side view of the tissue resection device of FIG. 6 with the protective hood in the first position; and
FIG. 9 is a close-up side view of this tissue resection device of FIG. 7 with the protective hood in the second position.

### DETAILED DESCRIPTION

Standard medical planes of reference and descriptive terminology are employed in this specification. A sagittal plane divides a body into right and left portions. A mid-sagittal plane divides the body into bilaterally symmetric right and left halves. A coronal plane divides a body into anterior and posterior portions. A transverse plane divides a body into superior and inferior portions. Anterior means toward the front of the body. Posterior means toward the back of the body. Superior means toward the head. Inferior means toward the feet. Medial means toward the midline of the body. Lateral means away from the midline of the body. Axial means toward a central axis of the body. Abaxial means away from a central axis of the body. Ipsilateral means on the same side of the body. Contralateral means on the opposite side of the body. These descriptive terms may be applied to an animate or inanimate body.

The following disclosure is made in the context of femoroacetabular impingement (FAI) surgery of the hip for the purposes of illustration. The principles of the disclosed technology are applicable to a variety of other tools and instruments beyond the scope of FAI.

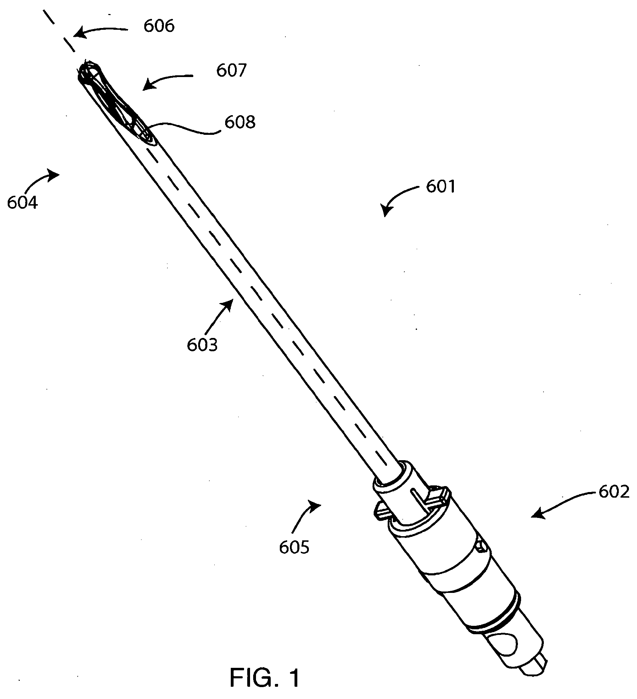

Referring to FIG. 1, a tissue resection device 601 may include a drive adapter hub 602, a hollow member 603, and a rotary cutting member 607. The hollow member 603 has a proximal end 605, a distal end 604, and a central axis 606. The drive adapter hub 602 is coupled to the hollow member 603 at the proximal end 605. The rotary cutting member 607 is disposed at the distal end 604 of the hollow member 603. The rotary cutting member 607 may be described as a burr. The tissue resection device 601 may also incorporate a suction port 608 to aid in removing tissue debris. In some examples, the drive adapter hub 602 may be adapted for grasping the tissue resection device and manipulating the position of the tissue resection device in any direction or orientation. For example, the drive adapter hub 602 may be adapted to interact with a powered hand piece (not shown) forming a handle for grasping the tissue resection device and manipulating the position of the tissue resection device in any direction or orientation. In other examples, the drive adapter hub 602 may include an integral handle formed thereon. In still other examples, the drive adapter hub 602 may be externally textured or may have a high friction outer surface for grasping.

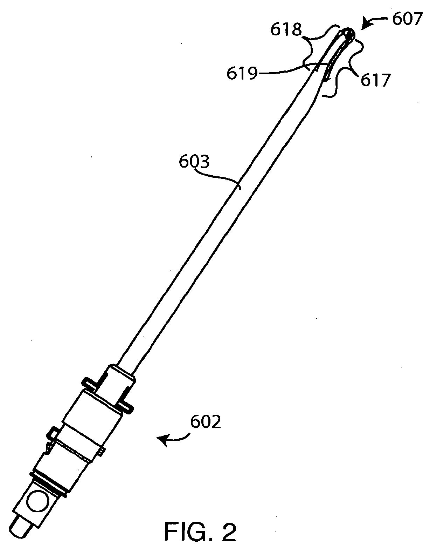

FIG. 2 shows the tissue resection device 601 from a side view. The hollow member 603 may have an opening or window 617 formed in the distal end 604 of the hollow member 603. The opening or window 617 may be formed along the axis 606 of the hollow member 603 and sized and shaped to allow the rotary cutting member 607 to be partially exposed and protrude from the window 617. The shape of the window 617 in the direction of the axis 606 of the hollow member 603 may have edges 619 that are concave shaped and substantially similar to the concave shape of the rotary cutting member 607. The rotary cutting member 607 may protrude uniformly past the edges 619. The concave shaped edges 619 of the window 617 may thus act as depth stops to limit the amount of tissue that is resected during a single pass of the tissue resection device 601. In one example, the rotary cutting member 607 protrudes about 1.5 mm past the concave shaped edges 619 of the window 617. In other examples, the rotary cutting member 607 protrudes less than 1.5 mm beyond the concave shaped edges 619 of the window 617. In still other examples, the rotary cutting member 607 protrudes more than 1.5 mm beyond the concave shaped edges 619 of the window 617. In some examples, the user may selectively adjust the amount that the rotary cutting member 607 protrudes from the concave shaped edges 619 of the window 617.

The concave shaped edges 619 may also give the user better control by contacting the tissue during resection on one or both sides of the rotary cutting member 607. The edges 619 may provide stability to the resection device 601 by resisting twisting forces caused by the rotary cutting member as it spins and resects tissue. The edges 619 may also provide stability to the resection device by providing a multi-contact stabilizing reference to help the user keep the burr or rotary cutting member 607 in the desired position, similar to a tripod or a tool rest. For example, there may be a first contact point or area between the burr 607 and the tissue as the burr 607 resects the tissue; a second contact point or area between the tissue and a first concave shaped edge 619, and a third contact point or area between the tissue and a second concave shaped edge 619 opposite the first concave shaped edge. Each additional contact point provides greater stability and control to the user.

A distal top portion 618 of the hollow member 603 may be entirely or partially formed of transparent material to allow the user to see the rotary cutting member 607 during use. In some examples, the rotary cutting member 607 may be fully exposed with no distal top portion 618 or protective hood 621 (discussed below) adjacent the rotary cutting member 607, or any window 617 with concave shaped edges 619 to act as depth stops.

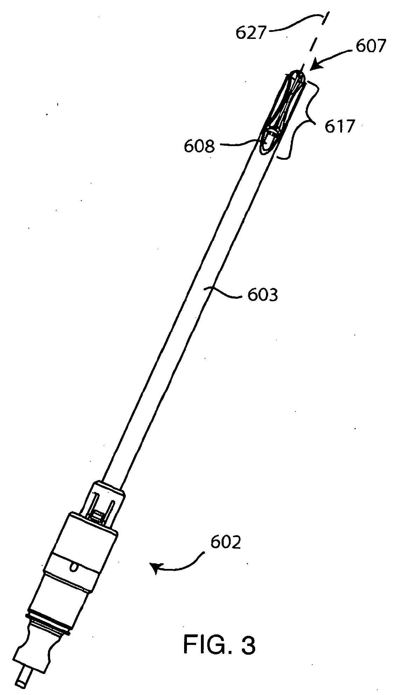

FIG. 3 shows a bottom view of the tissue resection device 601 illustrating the window 617 formed in the hollow member 603 and the concave or hourglass shaped rotary cutting member 607 exposed through the window 617. The rotary cutting member 607 may have an elongated body 609 that lies along an elongate body central longitudinal axis 627. In some examples, the rotary cutting member 607 may be coaxial with the axis 606 of the hollow member 603. In other examples, the axis 627 may be parallel to the axis 606.

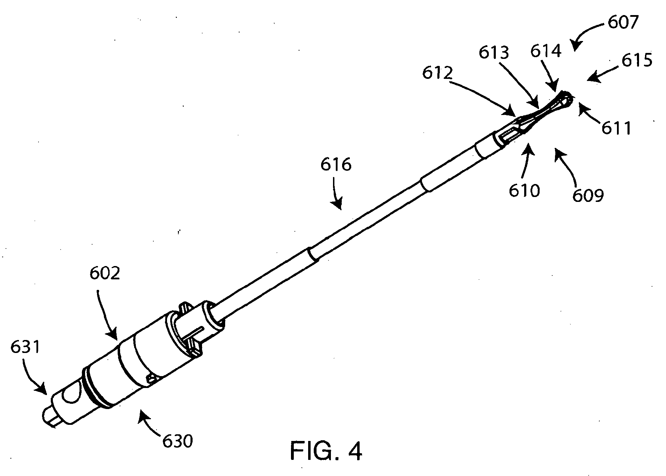

FIG. 4 shows the tissue resection device 601 with the hollow member 603 removed to expose a drive member 616. The drive member 616 may transmit rotational forces, or torque, from the hub 602. The hub 602 may transmit torque from a powered hand piece (not shown), which may be connected to a suitable power source (not shown). The hub 602 may include a drive feature 631, for example a triangular, rectangular, square, or hexagonal shaft, for complementary interconnection with the powered hand piece.

The rotary cutting member 607 may have one or more cutting features on the elongate body 609. FIG. 4 shows an example with cutting flutes along the length of the elongate body 609 of the rotary cutting member 607. The cutting flutes may be of any size, shape, or number. The cutting flutes may be axial as shown, or they may be helical clockwise or counterclockwise. The cutting flutes may cross to produce a diamond pattern of cutting projections. The rotary cutting member 607 may have a first portion 612, a second portion 614, a third or middle portion 613, and a fourth portion 615. The rotary cutting member 607 may have a concave or hourglass shape between the proximal end 610 and the distal end 611 of the rotary cutting member 607. The first portion 612 may have a first width or diameter, the second portion 614 may have a second width or diameter, and the middle portion 613 may have a third width or diameter. The first width of the first portion 612 and the second width of the second portion 614 may be greater than the third width of the middle portion 613 to form a concave curve between the first portion 612 and the second portion 614 of the rotary cutting member 607. The first, second, and third widths or diameters may be measured over the cutting features, and may thus correspond to the dimensions cut by the rotary cutting member 607. The concave shape of the rotary cutting member 607 may be chosen to substantially correspond to the natural or desired curvature of the tissue to be resected. For example, the concave shape of the rotary cutting member 607 may be chosen to substantially correspond to the natural curvature of the femoral neck of a human bone. In other examples, the concave shape of the rotary cutting member 607 may be chosen to substantially correspond to the natural or desired curvature of any tissue including, but not limited to, any bone, cartilage, soft tissue, and the like.

Continuing with FIG. 4 , rotary cutting member 607 may have a fourth portion or end cutter 615 formed on the distal end 611 of the elongate body 609 of the rotary cutting member 607. In some examples, the end cutter portion 615 is at least partially hemispherical in shape. In other examples, the end cutter portion 615 may be substantially flat, conical, dished, or another shape. The end cutter portion 615 may have flutes, cutting members, or abrasive material formed thereon and configured to resect tissue. For example, the end cutter portion 615 may be useful in removing pincer-type bone impingements on an acetabular rim during femoral acetabular impingement (FAI) surgery. In other examples, the rotary cutting member 607 may terminate in a smooth non-cutting end portion instead of an end cutter.

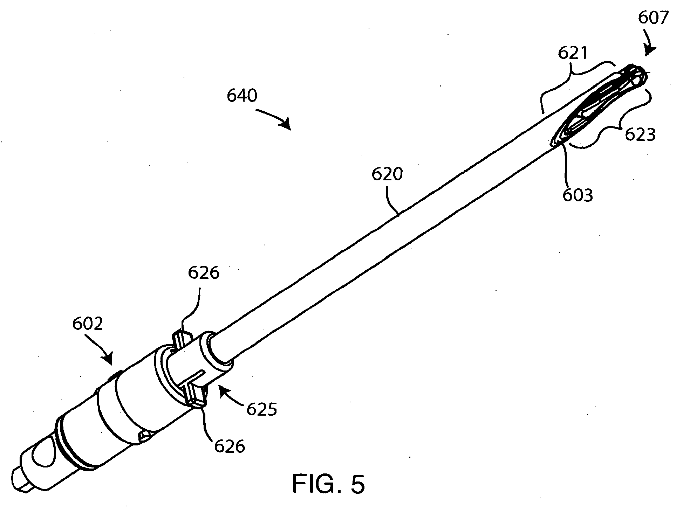

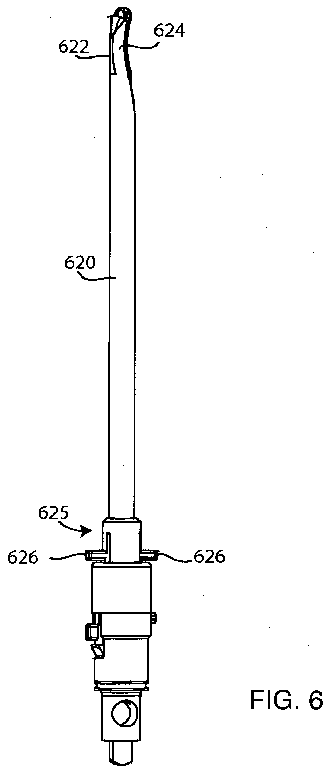

FIGS. 5-9 illustrate another tissue resection device 640 that is similar to device 601, but includes an outer sheath 620 having a protective hood 621 and a protective hood window 623 formed on the distal end of the outer sheath 620. In this example, the outer sheath 620 is a tubular structure that fits over a hollow member 603. The protective hood window 623 may be similar to window 617. The window 623 may be formed along the axis 606 of the hollow member 603 and sized and shaped to allow the rotary cutting member 607 to be partially exposed and protrude from the protective hood window 623 formed in the hollow member 603. The shape of the protective hood window 623 in the direction of the axis 606 of the hollow member 603 may have edges 624 that are concave and substantially similar to the concave shape of the rotary cutting member 607. The concave shaped edges 624 of the protective hood window 623 may thus act as depth stops to limit the amount of tissue that is resected during a single pass of the tissue resection device 640. The protective hood may be made of transparent material to help the user visualize the placement and action of the rotary cutting member 607. In one example, the outer sheath 620 may be rotated by manipulating the control member 625 located at a proximal end of the outer sheath 620. For example, a user may apply compressive and/or rotational forces to tabs 626 of the control member 625 to rotate the outer sheath 620 about the hollow member 603 and control the position of the protective hood 621 relative to the rotary cutting member 607. In this manner, the protective hood 621 may be selectively positioned to expose the rotary cutting member 607 to allow tissue to be resected, or positioned to cover the rotary cutting member 607 to prevent tissue from being resected, or positioned to partially cover the rotary cutting member 607.

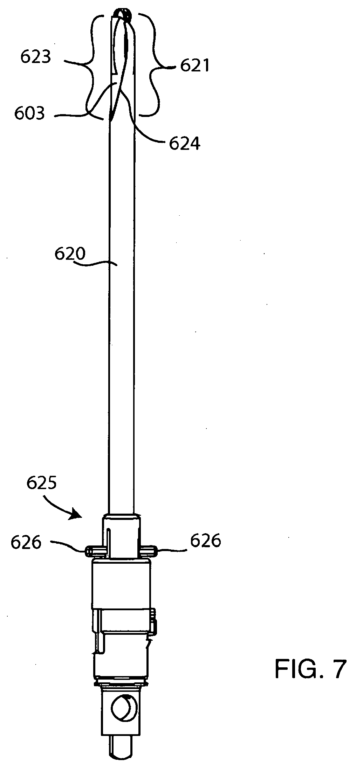

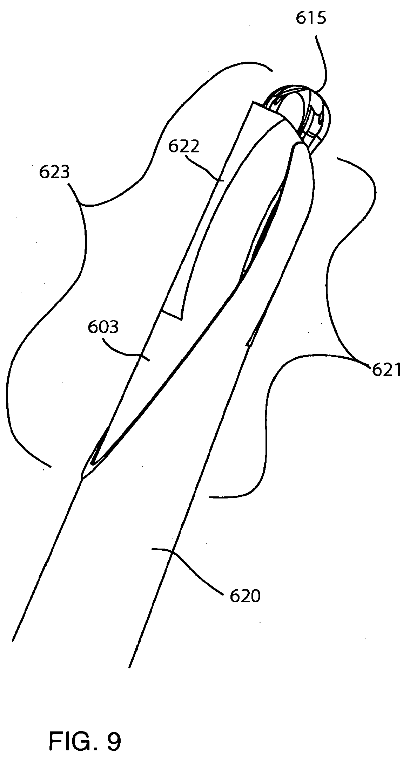

FIGS. 5, 6, and 7 show the tissue resection device 640 with the outer sheath 620 rotationally oriented such that the protective hood window 623 of the outer sheath 620 is aligned with the window 617 of the hollow member 603. This allows the rotary cutting member to be exposed through both windows 623, 627 and enables the rotary cutting member 607 to resect tissue. FIGS. 7 and 9 show the tissue resection device 640 with the outer sheath 620 rotationally oriented such that the protective hood window 623 of the outer sheath 620 faces the window 617 of the hollow member 603. This covers up the rotary cutting member 607 and prevents the rotary cutting member 607 from resecting tissue along the curved portion of the rotary cutting member 607. In some examples, the end-cutting portion 615 of the rotary cutting member 607 always remains exposed at the distal tip of the tissue resection device 640 regardless of the rotational orientation of the outer sheath 620. In other examples, the end-cutting portion 615 of the rotary cutting member 607 may be selectively covered or uncovered to allow or prevent the end-cutting portion 615 from resecting tissue. In one such example, the outer sheath 620 and the hollow member 603 may extend distally to cover some of the end-cutting portion 615. When the protective hood window 623 of the outer sheath 620 is aligned with the window 617 of the hollow member 603, some of the end-cutting portion 615 is exposed. When the protective hood window 623 of the outer sheath 620 faces the window 617 of the hollow member 603, the end-cutting portion 615 is covered up. In another such example, the outer sheath may be translatable along the axis 606 of the hollow member 603 to cover portions of the rotary cutting member 607. For example, the outer sheath 620 may be translated in the distal direction to substantially cover up all or a portion of the hourglass and/or end cutting portions 615 of the tissue resection device 640. Thus, it will be understood, that the outer sheath 620 may be both rotatably and axially positionable.

It is to be understood that a rotatable or translatable protective hood 621 may be used with any surgical instrument, besides burrs, to cover up the end effector disposed at the distal end of the hollow member 603. Example end effectors include, but are not limited to: grasping jaws, dissectors, suture passers, staplers, tissue cutters, rasps, ablation devices, microfracture devices, scalpels, shavers, cameras, sensors, etc., or any other end effector or structure.

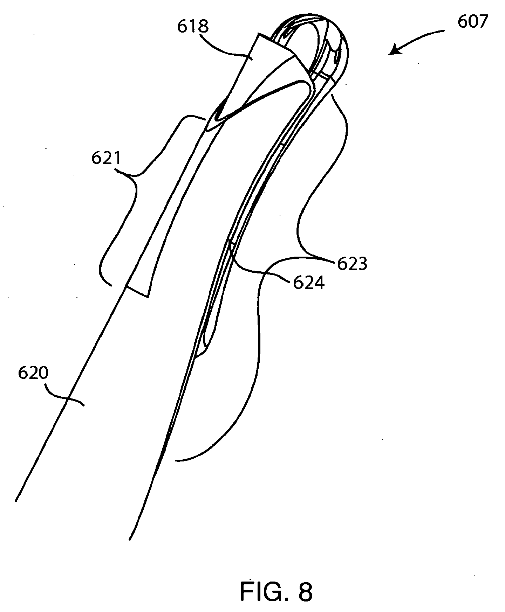

With reference to FIG. 8 , it may be seen that when the outer sheath 620 is rotated to the open position, the rotary cutting member 607 and the suction port are exposed through the window 623 and the suction created by suction port 608 is proximal to the rotary cutting member 607. Thus, suction power is positioned close to the curved portion of the rotary cutting member 607 where it is needed to remove tissue debris. In contrast, when the outer sheath 620 is rotated to the closed position, the protective hood 621 covers the curved portion of the rotary cutting member 607 and the suction port 608. This directs suction distally toward the end-cutting portion 615 of the rotary cutting member 607, as may be seen in FIG. 9. Thus, the design of the outer sheath 620 automatically shifts fluid suction between the proximal part of the rotary cutting member 607 to the distal end-cutting portion 615 of the rotary cutting member 607 as the outer sheath 620 is rotated.

Similarly, in other examples, the outer sheath 620 may be axially translatable to automatically direct fluid suction forces between the proximal part of the rotary cutting member 607 and the distal end-cutting portion 615 of the rotary cutting member as the outer sheath 620 translates back and forth in the axial direction.

The components disclosed herein may be fabricated from metals, alloys, polymers, plastics, ceramics, glasses, composite materials, or combinations thereof, including but not limited to: PEEK, titanium, titanium alloys, commercially pure titanium grade 2, ASTM F67, Nitinol, cobalt chrome, stainless steel, ultra high molecular weight polyethylene (UHMWPE), biocompatible materials, and biodegradable materials, among others. Different materials may be used for different parts. Different materials may be used within a single part. Any component disclosed herein may be colored, coded or otherwise marked to make it easier for a user to identify the type and size of the component, the setting, the function(s) of the component, and the like.

It should be understood that the present systems, kits, apparatuses, and methods are not intended to be limited to the particular forms disclosed. Rather, they are to cover all combinations, modifications, equivalents, and alternatives falling within the scope of the claims.

The claims are not to be interpreted as including means-plus- or step-plus-function limitations, unless such a limitation is explicitly recited in a given claim using the phrase(s) "means for" or "step for," respectively.

The term "coupled" is defined as connected, although not necessarily directly, and not necessarily mechanically.

The use of the word "a" or "an" when used in conjunction with the term "comprising" in the claims and/or the specification may mean "one," but it is also consistent with the meaning of "one or more" or "at least one." The term "about" means, in general, the stated value plus or minus 5%. The use of the term "or" in the claims is used to mean "and/or" unless explicitly indicated to refer to alternatives only or the alternative are mutually exclusive, although the disclosure supports a definition that refers to only alternatives and "and/or."

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a method or device that "comprises," "has," "includes" or "contains" one or more steps or elements, possesses those one or more steps or elements, but is not limited to possessing only those one or more elements. Likewise, a step of a method or an element of a device that "comprises," "has," "includes" or "contains" one or more features, possesses those one or more features, but is not limited to possessing only those one or more features. Furthermore, a device or structure that is configured in a certain way is configured in at least that way, but may also be configured in ways that are not listed.

In the foregoing Detailed Description, various features are grouped together in several examples for the purpose of streamlining the disclosure. This method of disclosure is not to be interpreted as reflecting an intention that the examples of the invention require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example.

## Claims

1. A tissue resection device, comprising:
a hollow member (603) having a proximal end (605), a distal end (604), and a longitudinal axis (606);
a drive member (616) situated at least partly within the hollow member (603);
a rotary cutting member (607) for side cutting, the rotary cutting member (607) having an elongate body (609) with a longitudinal axis, a proximal end, and a distal end, wherein the proximal end is coupled to the drive member (616) and wherein the elongate body (609) has an hourglass shape between the proximal end of the elongate body (609) and the distal end of the elongate body (609) along the longitudinal axis of the elongate body (609); and
an opening (617), formed in the distal end of the hollow member (603) along the longitudinal axis (606) of the hollow member (603), the opening (617) of the hollow member (603) sized and shaped to partially expose the rotary cutting member (607) through the opening (617) of the hollow member (603), wherein
the shape of the opening (617) in the direction of the axis (606) of the hollow member (603) has edges (619), that are concave shaped and are substantially similar to the hourglass shape of the rotary cutting member (607),
**characterized in that**
the rotary cutting member (607) partially protrudes from the opening (617) of the hollow member (603).

2. The tissue resection device of claim 1, wherein the rotary cutting member (607) is coaxial with and received within the hollow member (603).

3. The tissue resection device of claim 1, wherein the longitudinal axis of the rotary cutting member (607) is parallel to the longitudinal axis (606) of the hollow member (603), wherein the rotary cutting member (607) is received within the hollow member (603).

4. The tissue resection device of claim 1, wherein a distance that the rotary cutting member (607) protrudes from the opening of the hollow member (603) is selectively adjustable.

5. The tissue resection device of claim 1, wherein the elongate body (609) of the rotary cutting member (607) further comprises an end cutting portion (615) formed in the distal end of the elongate body (609) of the rotary cutting member (603).

6. The tissue resection device of claim 5, wherein the end cutting portion (615) is at least partially hemispherical in shape.

7. The tissue resection device of claim 1, further comprising a protective hood (621) adapted to partially surround the rotary cutting member (607).

8. The tissue resection device of claim 7, wherein the protective hood (621) comprises an opening formed in a distal end of the protective hood (621) along the longitudinal axis of the rotary cutting member (607), the opening of the protective hood (621) being sized and shaped to partially expose the rotary cutting member (607) through the opening of the protective hood (621), wherein the rotary cutter (607) partially protrudes from the opening of the protective hood (621), wherein the shape of the opening of the protective hood (621) along the longitudinal axis of the rotary cutting member (607) is concave and substantially similar to the hourglass shape of the rotary cutting member (607).

9. The tissue resection device of claim 7, wherein the protective hood (621) is rotatable about the longitudinal axis of the rotary cutting member (607) to selectively cover and
uncover the rotary cutting member (607).

10. The tissue resection device of claim 1, wherein at least a portion of the hollow member (603) surrounding the rotary cutting member (607) comprises a transparent material.

11. The tissue resection device of claim 1, wherein the rotary cutting member (607) comprises:
- a first portion having a first width;
- a second portion having a second width; and
- a middle portion having a third width, the middle portion intermediate the first portion and the second portion, wherein the first width of the first portion and the second width of the second portion are greater than the third width of the middle portion.

12. The tissue resection device of claim 7, wherein the protective hood (621) can be selectively translated along the longitudinal axis to cover or uncover the rotary cutting member (607).

13. The tissue resection device of claim 1, wherein opening (617) provides a depth stop.

14. The tissue resection device of claim 1, wherein the rotary cutting member (607) is a burr.

15. The tissue resection device of claim 1, wherein the rotary cutting member (607) is a rasp.

## Patentansprüche

1. Geweberesektionsvorrichtung, aufweisend:
ein hohles Element (603), das ein proximales Ende (605), ein distales Ende (604) und eine Längsachse (606) aufweist;
ein Antriebselement (616), das zumindest teilweise innerhalb des hohlen Elements (603) angeordnet ist;
ein Drehschneidelement (607) zum Seitenschneiden, wobei das Drehschneidelement (607) einen länglichen Körper (609) mit einer Längsachse, einem proximalen Ende und einem distalen Ende aufweist, wobei das proximale Ende an das Antriebselement (616) gekoppelt ist und wobei der längliche Körper (609) eine Sanduhrform zwischen dem proximalen Ende des länglichen Körpers (609) und dem distalen Ende des länglichen Körpers (609) entlang der Längsachse des länglichen Körpers (609) aufweist; und
eine Öffnung (617), die in dem distalen Ende des hohlen Elements (603) entlang der Längsachse (606) des hohlen Elements (603) gebildet ist, wobei die Öffnung (617) des hohlen Elements (603) bemessen ist und eine Form aufweist, um das Drehschneidelement (607) teilweise durch die Öffnung (617) des hohlen Elements (603) freizulegen, wobei
die Form der Öffnung (617) in der Richtung der Achse (606) des hohlen Elements (606) Kanten (619) aufweist, die eine konkave Form aufweisen und im Wesentlichen der Sanduhrform des Drehschneidelements (607) ähnlich sind, **dadurch gekennzeichnet, dass**
das Drehschneidelement (607) teilweise von der Öffnung (617) des hohlen Elements (603) hervorsteht.

2. Geweberesektionsvorrichtung nach Anspruch 1, wobei das Drehschneidelement (607) koaxial mit dem hohlen Element (603) und darin aufgenommen ist.

3. Geweberesektionsvorrichtung nach Anspruch 1, wobei die Längsachse des Drehschneidelements (607) parallel zur Längsachse (606) des hohlen Elements (603) ist, wobei das Drehschneidelement (607) innerhalb des hohlen Elements (603) aufgenommen ist.

4. Geweberesektionsvorrichtung nach Anspruch 1, wobei ein Abstand, mit dem das Drehschneidelement (607) von der Öffnung des hohlen Elements (603) hervorsteht, selektiv einstellbar ist.

5. Geweberesektionsvorrichtung nach Anspruch 1, wobei der längliche Körper (609) des Drehschneidelements (607) ferner einen Endschneideabschnitt (615) aufweist, der in dem distalen Ende des länglichen Körpers (609) des Drehschneidelements (603) gebildet ist.

6. Geweberesektionsvorrichtung nach Anspruch 5, wobei der Endschneideabschnitt (615) zumindest teilweise halbkugelförmig ist.

7. Geweberesektionsvorrichtung nach Anspruch 1, die ferner eine Schutzhaube (621) aufweist, die ausgebildet ist, um das Drehschneidelement (607) teilweise zu umgeben.

8. Geweberesektionsvorrichtung nach Anspruch 7, wobei die Schutzhaube (621) eine Öffnung aufweist, die in einem distalen Ende der Schutzhaube (621) entlang der Längsachse des Drehschneidelements (607) gebildet ist, wobei die Öffnung der Schutzhaube (621) bemessen ist und eine Form aufweist, um das Drehschneidelement (607) teilweise durch die Öffnung der Schutzhaube (621) freizulegen, wobei das Drehschneidelement (607) teilweise von der Öffnung der Schutzhaube (621) hervorsteht, wobei die Form der Öffnung der Schutzhaube (621) entlang der Längsachse des Drehschneidelements (607) konkav und im Wesentlichen der Sanduhrform des Drehschneidelements (607) ähnlich ist.

9. Geweberesektionsvorrichtung nach Anspruch 7, wobei die Schutzhaube (621) um die Längsachse des Drehschneidelements (607) drehbar ist, um das Drehschneidelement (607) selektiv abzudecken und freizulegen.

10. Geweberesektionsvorrichtung nach Anspruch 1, wobei zumindest ein Abschnitt des hohlen Elements (603), das das Drehschneidelement (607) umgibt, ein transparentes Material aufweist.

11. Geweberesektionsvorrichtung nach Anspruch 1, wobei das Drehschneidelement (607) aufweist:
- einen ersten Abschnitt, der eine erste Breite aufweist;
- einen zweiten Abschnitt, der eine zweite Breite aufweist; und
- einen mittleren Abschnitt, der eine dritte Breite aufweist, wobei der mittlere Abschnitt zwischen dem ersten Abschnitt und dem zweiten Abschnitt liegt, wobei die erste Breite des ersten Abschnitts und die zweite Breite des zweiten Abschnitts größer sind als die dritte Breite des mittleren Abschnitts.

12. Geweberesektionsvorrichtung nach Anspruch 7, wobei die Schutzhaube (621) selektiv entlang der Längsachse translatorisch bewegt werden kann, um das Drehschneidelement (607) selektiv abzudecken oder freizulegen.

13. Geweberesektionsvorrichtung nach Anspruch 1, wobei die Öffnung (617) einen Tiefenanschlag bereitstellt.

14. Geweberesektionsvorrichtung nach Anspruch 1, wobei das Drehschneidelement (607) ein Fräser ist.

15. Geweberesektionsvorrichtung nach Anspruch 1, wobei das Drehschneidelement (607) eine Raspel ist.

## Revendications

1. Dispositif de résection de tissus, comprenant :
un élément creux (603) ayant une extrémité proximale (605), une extrémité distale (604), et un axe longitudinal (606) ;
un élément d'entraînement (616) situé au moins partiellement à l'intérieur de l'élément creux (603) ;
un élément de coupe rotatif (607) pour une coupe latérale, l'élément de coupe rotatif (607) ayant un corps allongé (609) avec un axe longitudinal, une extrémité proximale, et une extrémité distale, dans lequel l'extrémité proximale est couplée à l'élément d'entraînement (616) et dans lequel le corps allongé (609) a une forme de sablier entre l'extrémité proximale du corps allongé (609) et l'extrémité distale du corps allongé (609) le long de l'axe longitudinal du corps allongé (609) ; et
une ouverture (617), formée dans l'extrémité distale de l'élément creux (603) le long de l'axe longitudinal (606) de l'élément creux (603), l'ouverture (617) de l'élément creux (603) étant dimensionnée et formée pour exposer partiellement l'élément de coupe rotatif (607) à travers l'ouverture (617) de l'élément creux (603), dans lequel
la forme de l'ouverture (617) dans la direction de l'axe (606) de l'élément creux (603) a des bords (619) qui sont formés de façon concave et sont essentiellement similaires à la forme de sablier de l'élément de coupe rotatif (607),
**caractérisé en ce que**
l'élément de coupe rotatif (607) dépasse partiellement de l'ouverture (617) de l'élément creux (603).

2. Dispositif de résection de tissus selon la revendication 1, dans lequel l'élément de coupe rotatif (607) est coaxial à l'élément creux (603) et est reçu à l'intérieur de ce dernier.

3. Dispositif de résection de tissus selon la revendication 1, dans lequel l'axe longitudinal de l'élément de coupe rotatif (607) est parallèle à l'axe longitudinal (606) de l'élément creux (603), dans lequel l'élément de coupe rotatif (607) est reçu à l'intérieur de l'élément creux (603).

4. Dispositif de résection de tissus selon la revendication 1, dans lequel il est possible de régler sélectivement une distance de saillie de l'élément de coupe rotatif (607) à partir de l'ouverture de l'élément creux (603).

5. Dispositif de résection de tissus selon la revendication 1, dans lequel le corps allongé (609) de l'élément de coupe rotatif (607) comprend également une portion de coupe d'extrémité (615) formée dans l'extrémité distale du corps allongé (609) de l'élément de coupe rotatif (607).

6. Dispositif de résection de tissus selon la revendication 5, dans lequel la portion de coupe d'extrémité (615) est au moins partiellement de forme hémisphérique.

7. Dispositif de résection de tissus selon la revendication 1, comprenant également un capot de protection (621) adapté pour entourer partiellement l'élément de coupe rotatif (607).

8. Dispositif de résection de tissus selon la revendication 7, dans lequel le capot de protection (621) comprend une ouverture formée dans une extrémité distale du capot de protection (621) le long de l'axe longitudinal de l'élément de coupe rotatif (607), l'ouverture du capot de protection (621) étant dimensionnée et formée pour exposer partiellement l'élément de coupe rotatif (607) à travers l'ouverture du capot de protection (621), dans lequel le couteau rotatif (607) dépasse partiellement de l'ouverture du capot de protection (621), dans lequel la forme de l'ouverture du capot de protection (621) le long de l'axe longitudinal de l'élément de coupe rotatif (607) est concave et essentiellement similaire à la forme de sablier de l'élément de coupe rotatif (607).

9. Dispositif de résection de tissus selon la revendication 7, dans lequel le capot de protection (621) peut tourner autour de l'axe longitudinal de l'élément de coupe rotatif (607) pour couvrir et découvrir sélectivement l'élément de coupe rotatif (607).

10. Dispositif de résection de tissus selon la revendication 1, dans lequel au moins une portion de l'élément creux (603) entourant l'élément de coupe rotatif (607) comprend un matériau transparent.

11. Dispositif de résection de tissus selon la revendication 1, dans lequel l'élément de coupe rotatif (607) comprend :
- une première portion ayant une première largeur ;
- une deuxième portion ayant une deuxième largeur ; et
- une portion médiane ayant une troisième largeur, la portion médiane étant intermédiaire entre la première portion et la deuxième portion, dans lequel la première largeur de la première portion et la deuxième largeur de la deuxième portion sont plus grandes que la troisième largeur de la portion médiane.

12. Dispositif de résection de tissus selon la revendication 7, dans lequel le capot de protection (621) peut être déplacé sélectivement suivant un mouvement de translation le long de l'axe longitudinal pour couvrir ou découvrir l'élément de coupe rotatif (607).

13. Dispositif de résection de tissus selon la revendication 1, dans lequel l'ouverture (617) fournit une butée de profondeur.

14. Dispositif de résection de tissus selon la revendication 1, dans lequel l'élément de coupe rotatif (607) est une fraise.

15. Dispositif de résection de tissus selon la revendication 1, dans lequel l'élément de coupe rotatif (607) est une râpe.
